# EUROPÄISCHE PATENTSCHRIFT

(11) **EP 3 476 372 B1**
(45) Veröffentlichungstag und Bekanntmachung des Hinweises auf die Patenterteilung: **19.07.2023**
(21) Anmeldenummer: 17020502.5
(22) Anmeldetag: 26.10.2017
(51) Int. Cl.: A61F 5/01, A61F 13/08, A61F 5/00, A61F 13/00

(54) **MEDIZINISCHES, INSBESONDERE ORTHOPÄDISCHES, HILFSMITTEL**
MEDICAL, IN PARTICULAR ORTHOPAEDIC, AID
MOYEN AUXILIAIRE MÉDICAL, EN PARTICULIER ORTHOPÉDIQUE

(43) Veröffentlichungstag der Anmeldung: 01.05.2019
(73) Patentinhaber: medi GmbH & Co. KG, 95448 Bayreuth (DE)
(72) Erfinder: Waldsich, Bettina, 95448 Bayreuth (DE)

(56) Entgegenhaltungen:
- EP-A1- 2 954 879
- DE-U1-202008 005 286
- US-A1- 2013 084 777
- US-A1- 2014 162 531

## Beschreibung

Die vorliegende Erfindung bezieht sich auf ein medizinisches, insbesondere orthopädisches, Hilfsmittel, wie zum Beispiel Bandage, Orthese oder kompressives Bekleidungsstück, insbesondere für Patienten mit Osteoporose, nach dem Oberbegriff des Anspruchs 1 sowie auf eine medizinische, insbesondere orthopädische, Einheit nach Anspruch 15.

Derartige medizinische, insbesondere orthopädische, Hilfsmittel dienen insbesondere zum Stützen, Entlasten, Ruhigstellen, Führen und/ oder Schützen von Gewebe und/ oder Körperteilen eines Patienten. Bandagen beispielweise werden speziell für verschiedene Körperteile, wie z.B. Knie-, Ellenbogen- Handgelenke gefertigt und werden bei Entzündungen, Verletzungen oder präventiv getragen, um die entsprechenden Gelenke zu schonen, zu unterstützen oder diese vor erneuter Verletzung oder zu hoher Beanspruchung zu schützen.

Kompressive Bekleidungsstücke, insbesondere Beinbekleidungsstücke, wie zum Beispiel Socken, Strümpfe oder Strumpfhosen, dienen insbesondere dazu gezielt Druck auf den Körper eines Patienten auszuüben. Ziel ist es ein geschädigte Venen- und/ oder Lymphsystem eines Patienten zu entlasten. Zur Ausbildung der genannten medizinischen Hilfsmittel, insbesondere von Bandagen und Kompressionsstrümpfen, werden bevorzugt sogenannte kompressive Gestricke verwendet, die mittels einer Flach- oder Rundstrickmaschine flach- oder rundgestrickt werden.

Orthesen werden zur Stabilisierung, Entlastung, Ruhigstellung, Führung und/ oder Korrektur von Gliedmaßen oder des Rumpfes von Patienten eingesetzt. Insbesondere letztgenanntes spielt bei der Behandlung von Osteoporose, bei der durch die Abnahme der Knochendichte es zu einer Deformierung der Wirbelsäule und somit zu Fehlstellungen der Zwischenwirbelgelenke, Fehlfunktionen der Sehnen, Bänder und Muskulatur kommt, was Behinderungen im Alltag zur Folge hat, eine wichtige Rolle.

Eine derartige Rückenorthese zum Aufrichten der Wirbelsäule ist beispielsweise aus der EP 1 284 695 B1 bekannt, die auch den nächstliegenden Stand der Technik darstellt.

Die bekannte Orthese erlaubt durch Anmodellieren einer speziellen Rückenschiene der Orthese an die gekrümmten Wirbelsäule, die sich also von hinten an den Rücken anlegt, eine Aufrichtung der Wirbelsäule und somit des Oberkörpers des Patienten. Die Orthese selbst ist hierzu als normales Kleidungsstück, insbesondere als orthopädisches Kleidungsstück mit einer oben oder unten geöffneten länglichen Tasche zur Aufnahme der eng, elastisch oder mit geringem Spiel in dieser gehaltenen, die Wirbelsäule abstützenden, steifen Schiene ausgebildet. Der Grundkörper der Orthese ist hierzu bevorzugt als orthopädisch indizierte einteilige Unterwäsche ausgebildet, welche aus einem Baumwoll- oder Kunstfasermaterial besteht und verschiedene Stretchzonen aufweist, die im angelegten Zustand eine Spannung aufweisen.

Des Weiteren sind Orthesen zur Behandlung von Osteoporose bekannt, die neben einer die Wirbelsäule abstützenden Schiene, an Stelle eines normalen Kleidungsstückes, Befestigungsgurte, die nach Art von Rucksackträgern geführt sind, aufweisen.

Ferner sind Funktionskleidungsstücke in Form von Büstenhaltern bekannt, wie beispielsweise in der US 2014/0162531 A1 gezeigt, welche ebenfalls eine stützende Wirkung aufweisen und somit orthopädische Vorteile mit sich bringen.

Neben der Behandlung von Osteoporse mittels Orthesen ist es ferner bekannt, mittels Vitamin D-reicher Arzneimittel Osteoporose vorzubeugen oder sie zu lindern. Magnesium und Vitamin D sind dafür verantwortlich, dass Calcium aus der Nahrung aufgenommen und in Knochen eingebaut wird. Calcium ist eines der wichtigsten Bausteine menschlicher Knochen. Damit das Calcium aber überhaupt in die Knochen gelangen kann, ist Vitamin D unverzichtbar.

Alle bisher bekannten medizinischen Hilfsmittel streben lediglich eine therapeutische Behandlung, insbesondere das Stützen, Entlasten, Ruhigstellen, Führen und/ oder Schützen von Gewebe und/ oder Körperteilen eines Patienten an. Die Vorbeugung der zu behandelnden Schmerzen bzw. Krankheiten wird nicht durch das medizinische Hilfsmittel unterstützt, sondern erfolgt, beispielsweise bei Osteoporose, durch die Einnahme von Arzneimittel, insbesondere von Vitamin D Präparaten.

Als nachteilig erweist sich hierbei, dass mit der Einnahme von Arzneimitteln diverse Unannehmlichkeiten verbunden sind. Zum einen müssen die Arzneimittel käuflich erworben werden, d.h. der Patient hat einen finanziellen und zeitlichen Aufwand. Zum anderen muss der Patient die Arzneimittel ständig zur Hand haben, da die Einnahme regelmäßig erfolgen muss.

Die natürliche Versorgung mit Vitamin D erfolgt grundsätzlich nicht über die Ernährung, sondern über die Sonne. Durch direkte Sonneneinstrahlung auf die Haut kann der Körper selbst Vitamin D herstellen. Durch die Bekleidung der menschlichen Körper mit lichtundurchlässigen Kleidungsstücken und medizinischen Hilfsmitteln, wie zum Beispiel Orthesen oder Bandagen, wird jedoch eine eigene Produktion von Vitamin D verhindert.

Folglich ist zur Produktion von Vitamin D eine Einnahme entsprechender Arzneimittel erforderlich.

Es sind zwar aus dem Stand der Technik, beispielsweise aus der EP 0 604 601 B1, sonnenlichtdurchlässige textile Materialen und Bekleidungsstücke bekannt, jedoch sind diese überwiegend dazu ausgebildet im Bademodenbereich eingesetzt zu werden.

Alternativ finden sie auch Verwendung bei Oberkörperbekleidungsstücken, wie beispielsweise in der DE 20 2008 005 286 U1 gezeigt. Sie dienen also nur einem Zweck, nämlich der Bräunung der Haut während Körperbereiche des Trägeres bedeckt sind. Sie sind nicht für die Verwendung bei medizinischen, insbesondere orthopädischen, Hilfsmitteln, insbesondere zum Stützen, Entlasten, Ruhigstellen, Führen und/ oder Schützen von Gewebe und/ oder Körperteilen eines Patienten, geeignet bzw. dazu ausgebildet.

Das bekannte Textilmaterial ist gegenüber der zur Bräunung der Haut führenden UV Strahlung im Wesentlichen durchlässig ausgebildet. Für die UV Durchlässigkeit ist die Maschenöffnungsweite, also die Größe der Maschenöffnung verantwortlich. Entscheidend hierbei sind der Fadendurchmesser und die Fadendichte. Um die Durchlässigkeit weiter zu verbessern wird ferner eine wabenartige Struktur mit Maschenöffnungsreihen vorgeschlagen, die sich in winkelversetzten Richtungen erstreckt. Zusätzlich wird vorgeschlagen, das Textilmaterial mit kontrastierenden Leuchtfarben zu bedrucken, so dass bei Auflage auf der Haut das Material undurchsichtig ist.

Des Weiteren ist beispielsweise aus der DE 197 24 518 A1 bekannt, dass mittels chemischer Bandfilter die UV Durchlässigkeit, insbesondere die Durchlässigkeit von UV-A und UV-B Strahlung variabel in den Sonnenschutzfasern, insbesondere in den Sonnenschutztextilien, einstellbar ist. Auch dieses Textilmaterial ist für den Einsatz als Sonnenbräunungskleidung ausgebildet.

Aus der US 2013/0084777 A1 ist ein Büstenhalter, insbesondere für den Sportbereich, bekannt, welcher besonders stabil ausgebildet ist und aus einem durchbräunenden Gewebe gefertigt ist. Das Gewebe weist hierzu sehr kleine Löcher auf, welche es erlauben, dass das UV Licht durch das Gewebe hindurchdringen kann.

Nicht bekannt aus dem Stand der Technik sind elastische und/ oder unelastische Textilmaterialien bzw. Kleidungsstücke, welche zum Stützen von Gewebe und/ oder Körperteilen eines Patienten, insbesondere zur Stabilisierung, Entlastung, Ruhigstellung, Führung und/ oder Korrektur von Gliedmaßen oder des Rumpfes von Patienten ausgebildet sind, insbesondere kompressive Gestricke, welche zusätzlich zu einer kompressiven Wirkung auch eine Durchlässigkeit gegenüber UV Strahlung aufweisen. An diese Textilmaterialen zur Erzeugung von medizinischer Kompression, werden andere Ansprüche gestellt, als an handelsübliche Textilmaterialien, welche u.a. für den Bademodebereich eingesetzt werden. Die Stütz- und Kompressionswirkung dieser Materialien ist um ein vielfaches höher.

Der vorliegenden Erfindung liegt die Aufgabe zugrunde ein medizinisches, insbesondere orthopädisches, Hilfsmittel zu schaffen, welches die Nachteile aus dem Stand der Technik vermeidet, insbesondere neben einem Stützen, Entlasten, Ruhigstellen, Führen und/ oder Schützen von Gewebe und/ oder Körperteilen eines Patienten, gleichzeitig zur Prävention von Vitamin D Mangel ausgebildet ist.

Gemäß einem Ausführungsbeispiel des medizinischen, insbesondere orthopädischen Hilfsmittel, wie zum Beispiel als Bandage, Orthese oder kompressives Bekleidungsstück, insbesondere für Patienten mit Osteoporose, besteht dieses aus zumindest einem Grundkörper aus zumindest einem elastischen und/ oder unelastischen Textilmaterial, welches zum Stützen von Gewebe und/ oder Körperteilen eines Patienten ausgebildet ist, sowie zumindest einem Funktionselement, das mit dem Grundkörper lösbar oder unlösbar verbindbar ist, wobei das zumindest eine Textilmaterial des Grundkörpers zusätzlich zur Prävention von Vitamin D Mangel zumindest teilweise, also zumindest bereichsweise, gegenüber UV Strahlung durchlässig ausgebildet ist.

Gemäß einem zweiten Ausführungsbeispiel des medizinischen, insbesondere orthopädischen Hilfsmittels ist das Textilmaterial des Grundkörpers gegenüber für die Vitamin D Produktion verantwortliche UV-B Strahlung von Sonnenlicht im Wesentlichen durchlässig und gegenüber der hautschädlichen UV-A Strahlung des Sonnenlichts im Wesentlichen undurchlässig ausgebildet.

Bevorzugt weist das Textilmaterial des Grundkörpers abschnittsweise unterschiedliche Durchlässigkeiten von UV Strahlung auf. Vorzugsweise umfasst der Grundkörper zumindest ein zweites Textilmaterial, das gegenüber UV-Strahlung weniger oder nicht durchlässig ausgebildet ist.

Gemäß einem dritten Ausführungsbeispiel ist das erste und/ oder das zumindest eine zweite Textilmaterial des Grundkörpers des medizinischen Hilfsmittels im Wesentlichen blickdicht ausgebildet. Hierdurch ist es möglich das Hilfsmittel gleichzeitig, also zusätzlich zur therapeutischen Behandlung, als Kleidungsstück zum Bedecken von Körperteilen zu tragen.

Das erste und/ oder das zumindest eine zweite Textilmaterial des Grundkörpers selbst ist bevorzugt aus einem Gestrick, Gewirk oder Gewebe gebildet. Im Falle einer Ausbildung des Textilmaterials als Gestrick handelt es sich bevorzugt um ein kompressives Strickteil, welches mittels einer Flach- oder Rundstrickmaschine gefertigt ist und einen Kompressionsfaden aufweist, der in ein Grundgestrick als Schussfaden oder maschenbildend eingearbeitet ist. Das Strickteil weist bevorzugt, durch die unterschiedliche Einbindung des Schussfadens in das Grundgestrick und/ oder durch die unterschiedliche Ausbildung des Grundgestricks, beispielsweise durch die unterschiedliche Anzahl von Maschen pro Maschenreihe, in Gestricklängsrichtung ein graduierten Kompressionsverlauf auf. Insbesondere bei der Ausbildung Grundgestricks als Kompressionsstrumpf, auch als Stützstrumpf bezeichnet, ist ein graduierter Kompressionsverlauf von Vorteil.

Damit das zumindest eine Textilmaterial, insbesondere das kompressive Gestrick, des Grundkörpers der Bandage, Orthese oder des kompressiven Bekleidungsstücks, gegenüber UV Strahlung durchlässig ist, wird vorgeschlagen, ein gegenüber UV Strahlung durchlässiges Garn, insbesondere Fasern, zu verwenden. Hierbei ist bevorzugt das Material des Garns, also die Fasern, gegenüber UV Strahlung durchlässig oder die Oberfläche des Garns bzw. der Fasern derart modifiziert, dass ein mit dem Garn hergestelltes Gestrick gegenüber UV Strahlung durchlässig ist. Für die Durchlässigkeit von UV Strahlung ist nämlich die Gewebekonstruktion, also die Zwischenräume des Garns, und/ oder die Transmission der UV-Strahlung durch die Faser, also die Faserart, verantwortlich. Bei Verwendung eines UV durchlässigen Materials wird insbesondere vorgeschlagen, das Garn, welches zur Erzeugung der kompressiven Wirkung bevorzugt aus einem hoch elastischen Fadenkern, insbesondere aus einem Elastan, besteht, welcher mit einem oder mehreren Fäden umwunden ist, gegenüber UV Strahlung durchlässig auszubilden. Hierbei ist insbesondere der zumindest eine Umwindefaden, insbesondere dessen Fasern, und/ oder der Fadenkern durchlässig gegenüber UV Strahlung. Bei diesen kompressiven Gestricken, also sehr dichten Gestricken, welche auch bevorzugt mehrlagig ausgebildet sind, ist die Ausbildung der UV Durchlässigkeit durch die Struktur, also durch die Maschenweite, wie sie aus dem Bademodenbereich mit sehr dünnen Textilien bekannt ist, eher ungeeignet, da die kompressive Wirkung des Gestricks dasselbe sehr stark zusammenzieht und dadurch sehr wenige Zwischenräume vorhanden sind. Alternativ kann die UV Durchlässigkeit natürlich auch durch die Kombination aus einer UV durchlässigen Gewebestruktur und einem oder mehreren UV durchlässigen Garnen erzielt werden.

Das Funktionselement ist bevorzugt ein Stützgurt, eine Pelotte, eine orthopädische Schiene, insbesondere Rückenschiene, oder eine Verbindungsvorrichtung zum lösbaren Positionieren des Grundkörpers an einem Patienten. Unter Verbindungsvorrichtung ist bevorzugt eine Steck-, Verriegelungs- oder eine Klettverschlussverbindung zu verstehen.

Gemäß einem weiteren Ausführungsbeispiel ist neben dem Textilmaterial des Grundkörpers auch das Funktionselement zur zusätzlichen therapeutischen Behandlung zumindest teilweise gegenüber UV-Strahlung im Wesentlichen durchlässig ausgebildet. Hierzu ist das Funktionselement bevorzugt aus einem gegenüber UV-Strahlung im Wesentlichen durchlässigen Material gefertigt. Alternativ ist auch eine konstruktive Lösung, beispielsweise ein poröser Aufbau des Funktionselements, möglich.

Zur Aufnahme des Funktionselements in dem Grundkörper des medizinischen Hilfsmittels, weist der Grundkörper zumindest eine Tasche auf, die derart ausgebildet ist, dass beispielsweise zumindest eine orthopädische Schiene darin aufnehmbar ist. Die Tasche ist hierzu bevorzugt auf der Innen- oder Außenseite des Grundkörpers aufgebracht, insbesondere aufgenäht oder aufgeschweißt, oder alternativ dazu in das Grundgestrick eingearbeitet, beispielsweise eingestrickt.

Gemäß einem weiteren Ausführungsbeispiel ist der Grundkörper socken- oder strumpfförmig ausgebildet. Er weist hierzu bevorzugt ein Gestrick, insbesondere ein kompressives Gestrick, auf. Der Grundkörper ist somit als Stumpf, insbesondere Wadenstrumpf oder Socke ausgebildet. Alternativ kann der socken- oder strumpfförmige Grundkörper auch Bestandteil einer Orthese oder Bandage, insbesondere Fußbandage, sein. Hierbei sind an dem Grundkörper bevorzugt ein oder mehrere Funktionselemente, wie zum Beispiel ein oder mehrere Stützgurte, Pelotten, Schienen und/ oder Verbindungsvorrichtungen befestigt.

Gemäß einem weiteren Ausführungsbeispiel ist der Grundkörper in Form einer einteiligen Unterwäsche, insbesondere als sogenannter Body, bevorzugt zur Behandlung von Osteoporose, ausgebildet. In Kombination mit einer speziellen Rückenschiene dient dieser dazu die Wirbelsäule und somit den Oberkörper eines Patienten aufzurichten. Der Grundkörper ist hierbei bevorzugt aus einem Gestrick oder einem Gewebe, wie zum Beispiel Baumwoll- oder Kunstfasermaterial, gefertigt und weist verschiedene Stretchzonen auf.

Gemäß einem Ausführungsbeispiel einer medizinischen, insbesondere orthopädischen, Einheit weist diese ein medizinisches, insbesondere orthopädisches, Hilfsmittel nach einem der vorherigen Ausführungsbeispiele sowie ein Bekleidungsstück, insbesondere Oberkörperbekleidungsstück oder Beinbekleidungsstück, auf, welches gegenüber UV-Strahlung, insbesondere gegenüber UV-B Strahlung, ebenfalls im Wesentlichen durchlässig ausgebildet ist. Das Oberkörperbekleidungsstück ist hierbei bevorzugt als Shirt, Langarmshirt oder beispielsweise als Pullover ausgebildet. Das Beinbekleidungsstück kann eine Hose, u.a. Strumpfhose, Hose mit kurzen oder langen Beinteilen, oder Badehose umfassen.

Das vorliegende medizinische Hilfsmittel zeichnet sich durch eine Reihe erheblicher Vorteile aus.

Durch die Ausbildung des Grundkörpers aus einem Textilmaterial, welches zumindest teilweise gegenüber UV Strahlung im Wesentlichen durchlässig ausgebildet ist, wird gleichzeitig neben einem Stützen, Entlasten, Ruhigstellen, Führen und/ oder Schützen von Gewebe und/ oder Körperteilen eines Patienten, eine Prävention von Vitamin D Mangel ermöglicht.

Durch die Durchlässigkeit von UV Strahlung, also der direkten Einstrahlung, insbesondere von Sonnenlicht, auf die Haut eines Patienten, kann der Körper selbst Vitamin D herstellen. Zur Produktion von Vitamin D ist also keine Einnahme entsprechender Arzneimittel erforderlich. Hierdurch ist der Anwender nicht dazu verpflichtet entsprechende Arzneimittel käuflich zu erwerben. Er hat also keinen weiteren, insbesondere zusätzlichen, finanziellen Aufwand. Im Gegenteil, er kann das kostenlose zur Verfügung stehende Sonnenlicht zur Produktion von Vitamin D nutzen.

Ein weiterer Vorteil der damit verbunden ist, ist der Wegfall der mit der Einnahme der Arzneimittel verbundene Zeitaufwand und das Erfordernis, die Mittel ständig zur Hand haben. Beides erleichtert die therapeutische Behandlung eines Patienten mit Beschwerden, insbesondere mit Osteoporose. Auch werden hochdosierte Vitamin D Kuren, die bei einem akuten Vitamin D Mangel notwendig sind, da übliche Dosierung nicht ausreichend sind, um einen Vitamin D Mangel schnell zu beheben, vermieden.

Einen weiteren Vorteil der Erfindung bildet die Möglichkeit gleichzeitig neben einem Stützen, Entlasten, Ruhigstellen, Führen und/ oder Schützen von Gewebe und/ oder Körperteilen eines Patienten, die Haut eines Patienten zur bräunen. Durch die Durchlässigkeit der natürlichen UV Strahlung, insbesondere der für die Bräunung der Haut verantwortlichen UV-B Strahlung des Sonnenlichts, tritt auch ein Bräunungseffekt an Stellen der Haut eines Patientenkörpers auf, an denen das erfindungsgemäße Hilfsmittel getragen wird. Das medizinische Hilfsmittel, kann somit insbesondere bei Freizeitaktivitäten, wie zum Beispiel Sonnenbaden, getragen werden, ohne unerwünschte Abzeichnungen an der Haut zu verursachen.

Nachfolgend wird die Erfindung anhand mehrerer Ausführungsbeispiele und in Verbindung mit den beigefügten Zeichnungen erläutert.

Dabei zeigt:
Figur 1 ein erstes Ausführungsbeispiel des medizinischen, insbesondere orthopädischen, Hilfsmittel in Form einer einteiligen Unterwäsche, insbesondere als sogenannter Body, zur Behandlung von Osteoporose,
Figur 2 ein zweites Ausführungsbeispiel des medizinischen, insbesondere orthopädischen, Hilfsmittel, in Form einer Bandage, insbesondere Kniebandage,
Figur 3 ein drittes Ausführungsbeispiel des medizinischen, insbesondere orthopädischen, Hilfsmittel, ausgebildet als Strumpf, insbesondere Kompressionsstrumpf, um ein geschädigte Venen- und/ oder Lymphsystem oder eine beanspruchte Muskelstruktur eines Patienten zu entlasten;

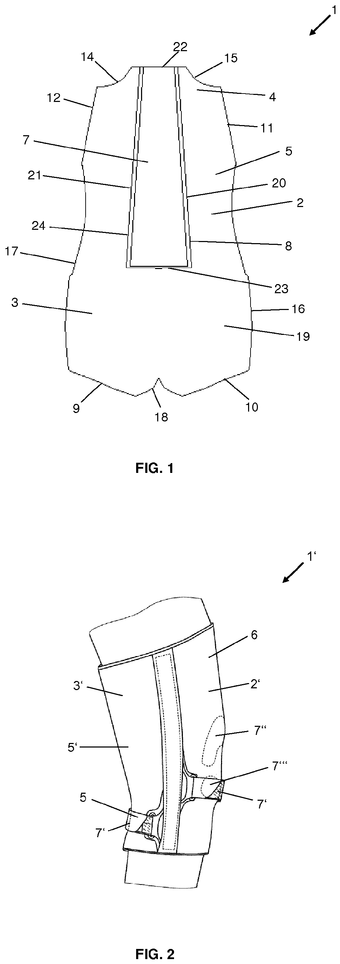

Das in Figur 1 schematisch dargestellte medizinische, insbesondere orthopädische, Hilfsmittel ist ein in Form einer einteiligen Unterwäsche ausgebildeter Body 1 zur Behandlung von Osteoporose. Dieser ist wie ein herkömmlicher Body geschnitten. Er weist mehrere Öffnungen, nämlich Öffnungen 9, 10 für die Beine, Öffnungen 11, 12 für die Arme sowie Öffnung 13 für den Kopf bzw. den Hals eines Patienten auf. An den Kanten 14, 15, 16, 17 und 18 ist der Body 1 geschlossen. Die Bereiche 14, 15 und 18 können alternativ auch zum Öffnen ausgebildet sein. An seiner Rückseite 19 weist der Body 1 eine entlang der Wirbelsäule verlaufende Tasche 8 auf, um ein Funktionselement 7, insbesondere eine orthopädische Schiene, die sich vorzugsweise von einem Steißbeinbereich, besondere bevorzugt von einem Kreuzbeinbereich, bis zu einem Halswirbelbereich erstreckt, aufzunehmen. Die Tasche 8 ist bevorzugt an den Seiten 20, 21 sowie am oberen Ende 22 geschlossen. Am unteren Ende 23 befindet sich eine Öffnung, durch die die Schiene 7 in der Tasche 8 aufnahmebar ist. Alternativ kann die Tasche 8 auch eine seitliche Öffnung aufweisen. Entsprechend der Schienenform, beispielsweise mit einer Verbreiterung 24 an deren unteren Ende, ist die Tasche 8 dieser angepasst. Um die sehr flache und passend an die Wirbelsäule des Patienten anmodellierte Schiene 7 an dem Body 1 in der Tasche 8 zu halten, kann hierzu ein Befestigungsmittel, beispielsweise ein Klettverschluss oder Druckknopf, bevorzugt an der Öffnung 23 der Tasche 8, vorgesehen. An der nicht gezeigten Vorderseite weist der Body 1, wie herkömmliche Bodys oder Jacken, eine Öffnung auf, welche mittels einem Reißverschluss oder mittels Druckknöpfen oder dergleichen verschließbar ist. Hierzu erstreckt sich die Öffnung bevorzugt mittig von einem oberen Ende des Bodys bis in einen unteren Bereich.

Des Weiteren umfasst der Body 1 zum Stützen und Entlasten von Gewebe und Körperteilen eines Patienten, insbesondere der Wirbelsäule, bevorzugt eine oder mehrere, insbesondere unterschiedlich stark ausgebildete, Stretchzonen, die durch unterschiedlich elastisches Textilmaterial gebildet sind, wobei zur Versteifung mehrere Textillagen übereinander vorhanden sein können. Alternativ kann der Body 1 auch aus einem, insbesondere kompressiven, Gestrick oder Gewirke gefertigt sein, welches zum Stützen von Gewebe und/ oder Körperteilen eines Patienten ausgebildet ist. Das kompressive Gestrick ist dadurch gekennzeichnet, dass es zusätzlich zu einem oder mehreren maschenbildenden Grundstrickfäden zumindest einen eingearbeiteten, bevorzugt eingelegten, Schussfaden enthält.

Das kompressive Gewebe oder Gestrick des Bodys 1 ist bevorzugt aus einem blickdichten Gewebe, gefertigt, welches gegenüber UV Strahlung im Wesentlichen durchlässig ausgebildet ist. Bevorzugt ist das Textilmaterial des Bodys 1 derart ausgebildet, dass es gegenüber für die Vitamin D Produktion verantwortliche UV-B Strahlung von Sonnenlicht im Wesentlichen durchlässig und gegenüber der hautschädlichen UV-A Strahlung des Sonnenlichts im Wesentlichen undurchlässig ist.

Das Funktionselement 7, nämlich die Rückenschiene, ist bevorzugt ebenfalls aus einem gegenüber UV-Strahlung im Wesentlichen durchlässigen Material, beispielsweise aus UV durchlässigem Acrylglas bzw. Plexiglas, gefertigt, um auf Grund der Durchlässigkeit der für die Bräunung der Haut verantwortlichen UV-B Strahlung des Sonnenlichts, unter anderem auch einen Bräunungseffekt an Stellen der Haut eines Patientenkörpers zu erzielen, an denen das Funktionselement 7 getragen wird.

Um insbesondere Sonnenbrände, auf Grund der Durchlässigkeit der für die Bräunung der Haut verantwortlichen UV-B Strahlung des Sonnenlichts des Bodys 1 an der Haut der Patienten zu vermeiden, kann der Body 1 des Weiteren bevorzugt abschnittsweise unterschiedliche Durchlässigkeiten von UV Strahlung aufweisen. Hierzu sind beispielsweise jene Bereiche des Bodys 1, die besonders der Sonnenstrahlung ausgesetzt sind, wie zum Beispiel die Bereiche, welche die Schultern des Anwenders bedecken, gegenüber UV-Strahlung weniger bis hin zu nicht durchlässig ausgebildet. Hierzu können diese Bereiche aus einem zweiten Textilmaterial 4 gefertigt sein, welches gegenüber UV Strahlung nicht durchlässig ausgebildet ist.

In Figur 2 ist ein zweites Ausführungsbeispiel des medizinischen, insbesondere orthopädischen, Hilfsmittels, in Form einer Bandage, insbesondere Kniebandage 1', gezeigt. Die Kniegelenkbandage 1' umfasst hierbei einen elastischen Grundkörper 2' mit einem in Tragestellung über die Patellasehne verlaufenden Funktionselement, insbesondere Pelotte 7". Um ausreichend viel Druck auf die Sehne ausüben zu können, weist die Kniegelenkbandage 1" neben der bevorzugt lösbar vom Grundkörper 2' ausgebildeten Pelotte 7" bevorzugt weitere Funktionselemente, nämlich Spanngurte 7' und Verbindungsvorrichtungen 7‴ auf. Die Spanngurte 7" sind bevorzugt unlösbar mit dem Grundkörper 2' verbunden. Ein erster Spanngurt verläuft vorderseitig nur abschnittsweise um den Grundkörper 2'. Ein zweiter Spanngurt ist rückseitig und höhenmäßig versetzt zum ersten Spanngurt an der Kniegelenkbandage 1' vorgesehen. Auch dieser Gurt verläuft nur abschnittsweise um den Grundkörper 2'.

Der Grundkörper 2' ist bevorzugt aus einem Gestrick 5', insbesondere einem blickdichten flach- oder rundgesrickten und kompressiven Gestrick, gefertigt, welches zum Stützen von Gewebe und/ oder Körperteilen eines Patienten und gegenüber UV Strahlung im Wesentlichen durchlässig ausgebildet ist. Bevorzugt ist auch das Gestrick 5' derart ausgebildet, dass es gegenüber für die Vitamin D Produktion verantwortliche UV-B Strahlung von Sonnenlicht im Wesentlichen durchlässig und gegenüber der hautschädlichen UV-A Strahlung des Sonnenlichts im Wesentlichen undurchlässig ist.

Die Funktionselemente, nämlich die Spanngurte 7' und die Pelotte 7" sind bevorzugt ebenfalls aus einem gegenüber UV-Strahlung im Wesentlichen durchlässigen Material gefertigt. Hierbei sind die Spanngurte 7' bevorzugt aus einem gegenüber UV Strahlung durchlässigen Gewebe 5 gefertigt. Die Pelotte 7" besteht bevorzugt aus einem UV durchlässigem Kunststoffkörper.

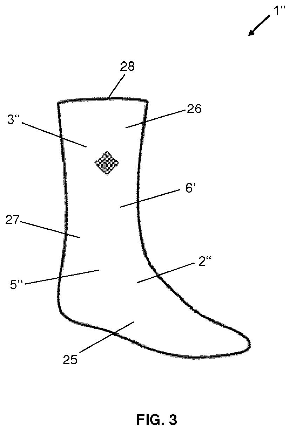

In Figur 3 ist das medizinische, insbesondere orthopädische, Hilfsmittel als Strumpf, insbesondere Kompressionsstrumpf 1‴, bestehend aus einem kompressiven Strickteil 5", ausgebildet, um ein geschädigte Venen- und/ oder Lymphsystem eines Patienten zu entlasten. Der in diesem Ausführungsbeispiel einstückig ausgebildete Kompressionsstrumpf 1" besteht aus einem Fußteil 25 sowie Wadenteil 26. Alternativ ist es auch denkbar, dass die Teile 25, 26 lösbar miteinander verbindbar sind. Das Fußteil 25 ist ausgebildet, die Sohle, den Spann, den Knöchel sowie die Fessel 27 eines Anwenders zu bedecken. Das Wadenteil 26 umgibt zumindest teilweise den Wadenbereich eines Anwenders, um beispielsweise Druck auf die Wadenmuskulaturpumpe auszuüben. Der Kompressionsstrumpf 1" ist bevorzugt an Hand der Kompressionswerte des socken- bzw. strumpfförmigen Grundkörpers 2" im Fesselbereich 27 einer der normierten vier Kompressionsklassen zugeordnet. Hierzu weist der Strumpf 1" bevorzugt in jeder zweiten gestrickten Maschenreihe einen Schussfaden auf. Im Fesselbereich weist der Strumpf 1" die höchsten Kompressionswerte auf. In Richtung des oberen Ende 28 des Wadenteils 26 weist der Kompressionsstrumpf 1" einen graduierten, insbesondere abfallenden, Kompressionsverlauf auf. Der Strumpf 1" selbst besteht bevorzugt aus einem Strickteil 5", insbesondere aus einem kompressiven Strickteil 6', das vorzugsweise aus zumindest einem maschenbildenenden Grundstrickfaden sowie zumindest einem Kompressionsfaden, insbesondere eingelegten Schussfaden gebildet ist.

Auch in diesem Ausführungsbeispiel ist der Grundkörper 2", nämlich der Strumpf 2", aus einem gegenüber UV Strahlung im Wesentlichen durchlässig Textilmaterial gefertigt. Das kompressive Strickteil 6' ist hierbei zumindest teilweise, also zumindest an einer oder mehreren Stellen des Strumpfes, gegenüber UV Strahlung im wesentlichen durchlässig. Hierzu wird vorgeschlagen die Maschengröße oder die Struktur des Strickteils 6' derart auszubilden, dass das kompressive Strickteil 6' im Wesentlichen durchlässig für UV Strahlung ist. Alternativ kann auch, wie aus dem Stand der Technik bekannt, geeignetes Fadenmaterial verwendet werden, welches zusätzlich gegenüber für die Vitamin D Produktion verantwortliche UV-B Strahlung von Sonnenlicht im Wesentlichen durchlässig und gegenüber der hautschädlichen UV-A Strahlung des Sonnenlichts im Wesentlichen undurchlässig ist. Auch in diesem Ausführungsbeispiel ist der Grundkörper 2" im Wesentlichen blickdicht. Alternativ kann dieser jedoch auch, zumindest bereichsweise, transparent ausgebildet sein.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Medizinisches, insbesondere orthopädisches, Hilfsmittel (1, 1', 1"), wie zum Beispiel Orthese (1), Bandage (1') oder kompressives Bekleidungsstück (1"), bestehend aus zumindest einem Grundkörper (2, 2', 2") aus zumindest einem elastischen und/ oder unelastischen Textilmaterial (3, 3', 3"), welches zum Stützen von Gewebe und/ oder Körperteilen eines Patienten ausgebildet ist, sowie zumindest einem Funktionselement (7, 7', 7", 7‴), das mit dem Grundkörper (2, 2', 2") lösbar oder unlösbar verbindbar ist, **dadurch gekennzeichnet, dass** das zumindest eine Textilmaterial (3, 3', 3") des Grundkörpers (2, 2', 2") zumindest teilweise gegenüber UV Strahlung im Wesentlichen durchlässig ausgebildet ist.

2. Medizinisches, insbesondere orthopädisches, Hilfsmittel (1, 1', 1") nach Anspruch 1, **dadurch gekennzeichnet, dass** das Textilmaterial (3, 3', 3") des Grundkörpers (2, 2', 2") gegenüber für die Vitamin D Produktion verantwortliche UV-B Strahlung von Sonnenlicht im Wesentlichen durchlässig und gegenüber der hautschädlichen UV-A Strahlung des Sonnenlichts im Wesentlichen undurchlässig ausgebildet ist.

3. Medizinisches, insbesondere orthopädisches, Hilfsmittel (1) nach einem der vorherigen Ansprüche, **dadurch gekennzeichnet, dass** das Textilmaterial (3) des Grundkörpers (2) abschnittsweise unterschiedliche Durchlässigkeiten von UV Strahlung aufweist.

4. Medizinisches, insbesondere orthopädisches, Hilfsmittel (1) nach einem der vorherigen Ansprüche, **dadurch gekennzeichnet, dass** der Grundkörper (2) zumindest ein zweites Textilmaterial (4) umfasst, das gegenüber UV-Strahlung weniger oder nicht durchlässig ausgebildet ist.

5. Medizinisches, insbesondere orthopädisches, Hilfsmittel (1, 1', 1") nach einem der vorherigen Ansprüche, **dadurch gekennzeichnet, dass** das erste und/ oder das zumindest eine zweite Textilmaterial (3, 3', 3"; 4) des Grundkörpers (2, 2', 2") im Wesentlichen blickdicht ausgebildet ist.

6. Medizinisches, insbesondere orthopädisches, Hilfsmittel (1, 1', 1") nach einem der vorherigen Ansprüche, **dadurch gekennzeichnet, dass** das erste und/ oder das zumindest eine zweite Textilmaterial (3, 3', 3"; 4) des Grundkörpers (2, 2', 2") aus einem Gewebe (5), Gestrick (5', 5"), oder Gewirke gebildet ist.

7. Medizinisches, insbesondere orthopädisches, Hilfsmittel (1',1") nach Anspruch 6, **dadurch gekennzeichnet, dass** das Gestrick (5', 5") ein kompressives Strickteil (6, 6') ist.

8. Medizinisches, insbesondere orthopädisches, Hilfsmittel (1") nach Anspruch 7, **dadurch gekennzeichnet, dass** das kompressive Strickteil (6') einen graduierten Kompressionsverlauf aufweist.

9. Medizinisches, insbesondere orthopädisches, Hilfsmittel (1, 1', 1") nach einem der vorherigen Ansprüche, **dadurch gekennzeichnet, dass** das Funktionselement (7, 7', 7", 7‴) gegenüber UV-Strahlung im Wesentlichen durchlässig ausgebildet ist.

10. Medizinisches, insbesondere orthopädisches, Hilfsmittel (1, 1', 1") nach einem der vorherigen Ansprüche, **dadurch gekennzeichnet, dass** das Funktionselement eine orthopädische Schiene (7), insbesondere Rückenschiene, ein Stützgurt (7'), eine Pelotte (7"), oder eine Verbindungsvorrichtung (7‴) zum lösbaren Positionieren des Grundkörpers (2') an einem Patienten ist.

11. Medizinisches, insbesondere orthopädisches, Hilfsmittel (1) nach einem der vorherigen Ansprüche, **dadurch gekennzeichnet, dass** der Grundkörper (2) zumindest eine Tasche (8) aufweist, die derart ausgebildet ist, dass zumindest eine orthopädische Schiene (8) darin aufnehmbar ist.

12. Medizinisches, insbesondere orthopädisches, Hilfsmittel (1") nach einem der vorherigen Ansprüche, **dadurch gekennzeichnet, dass** der Grundkörper (2") socken- oder strumpfförmig ausgebildet ist.

13. Medizinisches, insbesondere orthopädisches, Hilfsmittel (1) nach einem der vorherigen Ansprüche, **dadurch gekennzeichnet, dass** der Grundkörper (2) in Form einer einteiligen Unterwäsche, insbesondere als sogenannter Body, ausgebildet ist.

14. Medizinische, insbesondere orthopädische, Einheit aufweisend, ein medizinisches, insbesondere orthopädisches, Hilfsmittel (1, 1', 1") nach einem der vorherigen Ansprüche und ein Bekleidungsstücks, insbesondere Oberkörperbekleidungsstück oder Beinbekleidungsstück, welches gegenüber UV-Strahlung, insbesondere gegenüber UV-B Strahlung, im Wesentlichen durchlässig ausgebildet ist.

## Claims

1. Medical, in particular orthopaedic, aid (1, 1', 1") such as, for example orthosis (1), bandage (1') or compressive garment (1") consisting of at least one base body (2, 2', 2") formed of at least one elastic and/or non-elastic textile material (3, 3', 3") which is configured to support tissue and/or body parts of a patient, and at least one functional element (7, 7', 7", 7"') which can be connected detachably or non-detachably to the base body (2, 2', 2"), **characterized in that** the at least one textile material (3, 3', 3") of the base body (2, 2', 2") is configured to be at least partially substantially transmissible with respect to UV radiation.

2. Medical, in particular orthopaedic, aid (1, 1', 1") according to Claim 1, **characterized in that** the textile material (3, 3', 3") of the base body (2, 2', 2") is configured to be substantially transmissible with respect to UV-B radiation of sunlight which is responsible for the production of vitamin D and to be substantially non-transmissible with respect to the UV-A radiation of sunlight which is harmful to the skin.

3. Medical, in particular orthopaedic, aid (1) according to one of the preceding claims, **characterized in that** the textile material (3) of the base body (2) has different transmissibilities of UV radiation in sections.

4. Medical, in particular orthopaedic, aid (1) according to one of the preceding claims, **characterized in that** the base body (2) comprises at least one second textile material (4) which is configured to be less or non-transmissible with respect to UV radiation.

5. Medical, in particular orthopaedic, aid (1, 1', 1") according to one of the preceding claims, **characterized in that** the first and/or the at least one second textile material (3, 3', 3"; 4) of the base body (2, 2', 2") is configured to be substantially opaque.

6. Medical, in particular orthopaedic, aid (1, 1', 1") according to one of the preceding claims, **characterized in that** the first and/or the at least one second textile material (3, 3', 3"; 4) of the base body (2, 2', 2") is formed from a woven fabric (5), knitted fabric (5', 5") or warp-knitted fabric.

7. Medical, in particular orthopaedic, aid (1', 1") according to Claim 6, **characterized in that** the knitted fabric (5', 5") is a compressive knitted part (6, 6').

8. Medical, in particular orthopaedic, aid (1") according to Claim 7, **characterized in that** the compressive knitted part (6') has a graduated compression profile.

9. Medical, in particular orthopaedic, aid (1, 1', 1") according to one of the preceding claims, **characterized in that** the functional element (7, 7', 7", 7"') is configured to be substantially transmissible with respect to UV radiation.

10. Medical, in particular orthopaedic, aid (1, 1', 1") according to one of the preceding claims, **characterized in that** the functional element is an orthopaedic splint (7), in particular back splint, a support strap (7'), a truss pad (7") or a connecting device (7"') for detachable positioning of the base body (2') on a patient.

11. Medical, in particular orthopaedic, aid (1) according to one of the preceding claims, **characterized in that** the base body (2) has at least one pocket (8) which is configured in such a manner that at least one orthopaedic split (8) can be received therein

12. Medical, in particular orthopaedic, aid (1") according to one of the preceding claims, **characterized in that** the base body (2") is configured to be sock- or stocking-shaped.

13. Medical, in particular orthopaedic, aid (1) according to one of the preceding claims, **characterized in that** the base body (2) is configured in the form of a one-part undergarment, in particular as a so-called body suit.

14. Medical, in particular orthopaedic, unit comprising a medical, in particular orthopaedic, aid (1, 1', 1") according to one of the preceding claims and an item of clothing, in particular an upper body garment or leg covering item which is configured to be substantially transmissible with respect to UV radiation, in particular with respect to UV-B radiation.

## Revendications

1. Dispositif médical (1, 1', 1"), notamment orthopédique, comme par exemple une orthèse (1), un bandage (1') ou un vêtement compressif (1"), composé d'au moins un corps de base (2; 2', 2") en au moins une matière textile (3, 3', 3") élastique ou non élastique, qui est conçu pour soutenir des tissus et / ou des parties corporelles d'un patient, ainsi que d'au moins un élément fonctionnel (7, 7', 7", 7"'), qui est susceptible d'être assemblé de manière amovible ou inamovible avec le corps de base (2, 2', 2"), **caractérisé en ce que** l'au moins une matière textile (3, 3', 3") du corps de base (2, 2', 2") est conçue en étant au moins partiellement perméable au rayonnement UV.

2. Dispositif médical (1, 1', 1"), notamment orthopédique selon la revendication 1, **caractérisé en ce que** la matière textile (3, 3', 3") du corps de base (2, 2', 2") est conçue en étant majoritairement perméable au rayonnement UVB de la lumière solaire, responsable pour la production de la vitamine D et en étant majoritairement imperméable au rayonnement UVA, nocif pour la peau de la lumière solaire.

3. Dispositif médical (1), notamment orthopédique selon l'une quelconque des revendications précédentes, **caractérisé en ce que** la matière textile (3) du corps de base (2) fait preuve par sections de perméabilités différentes au rayonnement UV.

4. Dispositif médical (1), notamment orthopédique selon l'une quelconque des revendications précédentes, **caractérisé en ce que** le corps de base (2) comprend au moins une deuxième matière textile (4), qui est conçue en étant moins ou non perméable au rayonnement UV.

5. Dispositif médical (1, 1', 1"), notamment orthopédique selon l'une quelconque des revendications précédentes, **caractérisé en ce que** la première et / ou l'au moins une deuxième matière textile (3, 3', 3" ; 4) du corps de base (2, 2', 2") est conçue en étant majoritairement opaque.

6. Dispositif médical (1, 1', 1"), notamment orthopédique selon l'une quelconque des revendications précédentes, **caractérisé en ce que** la première et / ou l'au moins une deuxième matière textile (3, 3', 3" ; 4) du corps de base (2, 2', 2") est conçue en un tissu (5), en un tricot (5', 5"), ou en un tissu en mailles.

7. Dispositif médical (1',1"), notamment orthopédique selon la revendication 6, **caractérisé en ce que** le tricot (5', 5") est une pièce tricotée (6, 6') compressive.

8. Dispositif médical (1"), notamment orthopédique selon la revendication 7, **caractérisé en ce que** la pièce tricotée (6') compressive fait preuve d'une courbe de compression graduée.

9. Dispositif médical (1, 1', 1"), notamment orthopédique selon l'une quelconque des revendications précédentes, **caractérisé en ce que** l'élément fonctionnel (7, 7', 7", 7‴) est conçu en étant majoritairement perméable au rayonnement UV.

10. Dispositif médical (1, 1', 1"), notamment orthopédique selon l'une quelconque des revendications précédentes, **caractérisé en ce que** l'élément fonctionnel est une armature orthopédique (7), notamment une armature dorsale, une ceinture de soutien (7'), un insert (7"), ou un dispositif d'assemblage (7‴) pour le positionnement amovible du corps de base (2') sur un patient.

11. Dispositif médical (1), notamment orthopédique selon l'une quelconque des revendications précédentes, **caractérisé en ce que** le corps de base (2) comporte au moins une poche (8), qui est conçue de telle sorte, qu'elle puisse recevoir au moins une armature (8) orthopédique.

12. Dispositif médical (1"), notamment orthopédique selon l'une quelconque des revendications précédentes, **caractérisé en ce que** le corps de base (2") est conçu en forme de chaussette ou en forme de bas.

13. Dispositif médical (1), notamment orthopédique selon l'une quelconque des revendications précédentes, **caractérisé en ce que** le corps de base (2) est conçu sous la forme d'un sous-vêtement d'une seule pièce, notamment d'un dénommé body.

14. Unité médicale, notamment orthopédique, comportant un dispositif (1, 1', 1") médical, notamment orthopédique selon l'une quelconque des revendications précédentes et un vêtement, notamment un vêtement couvrant la partie supérieure du corps ou un vêtement couvrant les jambes, lequel est conçu en étant majoritairement perméable au rayonnement UV, notamment au rayonnement UVB.
